Europäisches Patentamt

European Patent Office

Office européen des brevets

(11) Publication number: **0 216 607**
A2

# EUROPEAN PATENT APPLICATION

(21) Application number: 86307209.6

(22) Date of filing: 19.09.86

(51) Int. Cl.⁴: **C 12 P 1/02**
C 12 N 1/20, A 61 K 35/66
//(C12P1/02,C12R1:68,
C12N1:20,C12R1:68)

(30) Priority: 19.09.85 JP 205278/85

(43) Date of publication of application:
01.04.87 Bulletin 87/14

(84) Designated Contracting States:
AT BE CH DE FR GB IT LI LU NL SE

(71) Applicant: TAISHO PHARMACEUTICAL CO. LTD
24-1 Takata 3-chome Toshima-ku
Tokyo 171 (JP)

(72) Inventor: Mizoue, Kazutoshi
Minamiurawa-Dai-5-Rolaru-Kopo 107 Buzo 1-chome
Urawa-shi Saitama (JP)

Okazaki, Tadayasu
No. 1525-4 Mimuro
Urawa-shi Saitama (JP)

Hanada, Kazunori
No. 523-12 Haraichi
Ageo-shi Saitama (JP)

Amamoto, Toshiro
Kitabukuro-Jutaku 302 2-76-2, Kitabukuro
Ohmiya-shi Saitama (JP)

Yamagishi, Michio
No. 112-36 Owada Yoshimi-cho
Hiki-gun Saitama (JP)

Omura, Sadafumi
No. 523-7 Haraichi
Ageo-shi Saitama (JP)

(74) Representative: Carpenter, David et al
MARKS & CLERK Alpha Tower Suffolk Street Queensway
Birmingham B1 1TT (GB)

(54) **Physiologically active substance FD-838 and process for preparing the same.**

(57) A physiologically active substance FD-838 having the following physiochemical properties: (1) appearance: pale green powder: (2) melting point: 98 to 99°C; (3) elemental analysis: C 64.20%, H 5.36%; N 3.39%; (4) molecular weight: high-resolution mass spectrum - found: 411.1356, calculated: 411.1318; (5) molecular formula: $C_{22}H_{21}NO_7$; (6) $[\alpha]$ : 0° (C=0.1 in methanol sol.); (7) UV absorption: measured in a methanol solution, 252 nm ($\varepsilon$ 7500), 283 nm ($\varepsilon$ 4110), 336 nm ($\varepsilon$ 10007); (8) IR absorption spectrum: as shown in Fig. 1 (measured in chloroform sol.); (9) $^1$H-NMR spectrum: as shown in Fig. 2 (measured in $CDCl_3$ at 400 MHz); (10) $^{13}$C-NMR spectrum: as shown in Fig. 3 (measured in $CDCl_3$ at 100 MHz); (11) solubility: soluble in chloroform, benzene, methanol, ethanol, acetone and ethyl acetate; poorly soluble in ethyl ether, n-hexane and petroleum ether; insoluble in water; (12) color reaction: positive with sulfuric acid, iodide, anisaldehyde-$H_2SO_4$ and vaniline-$H_2SO_4$; negative with ninhydrin and BCG; (13) nature: basic (neither acidic nor neutral). A process for preparing a physiologically active substance FD-838 which comprises cultivating a physiologically active substance FD-838 producing microorganism of genus Aspergillus, and collecting said physiologically active substance FD-838 that has accumulated in the culture of said microorganism. The physiological active substance FD-838 can induce differentiation of leukemic cells in culture and inhibit growth of certain Gram-positive bacteria and true fungi.

## Description

### PHYSIOLOGICALLY ACTIVE SUBSTANCE FD-838 AND PROCESS FOR PREPARING THE SAME

### FIELD OF THE INVENTION

The present invention relates to a physiologically active substance FD-838 and a process for preparing the same.

### BACKGROUND OF THE INVENTION

The physiologically active substance FD-838 of the present invention is a novel substance having a molecular weight of about 411 and the molecular formula $C_{22}H_{21}NO_7$. None of the existing substances so far reported has the same physiochemical properties or physiological activities.

The present inventors have made concerted efforts to obtain a novel physiologically active substance having differentiation-inducing and carcinostatic capabilities from microorganisms isolated from the soil. As a result of their efforts, the present inventors found that a particular microorganism discovered by them produces a novel physiologically active substance having the ability to induce the differentiation of leukemic cells. The present invention has been accomplished on the basis of this finding. The present inventors named this novel physiologically active substance "a physiologically active substance FD-838".

### SUMMARY OF THE INVENTION

The microorganism that produces the end substance which is one of the essential requirements for attaining the object of the present invention was newly isolated from a soil sample collected at Kakunodate-machi, Senboku-gun, Akita, Japan. This microorganism was named "Aspergillus fumigatus Fresenius F-838" by the present inventors and has been deposited at the Fermentation Research Institute, the Agency of Industrial Science and Technology, Ministry of International Trade and Industry, Japan, under Deposition Number FERM P-8406 (conformity with the International Accession Number BP-1124 under Budapest Treaty). The aforesaid Fermentation Research Institute, Agency of Industrial Science and Technology, which is entitled to an international depository authority, is situated at 1-3, Higashi 1-chome, Yatabe-machi, Tsukuba-gun, Ibaraki-ken, Japan (postal no.: 305). The end substance of the present invention that is obtained by cultivating this strain is the physiologically active substance FD-838.

### BRIEF DESCRIPTION OF THE DRAWINGS

Fig. 1 shows the IR absorption spectrum of the physiologically active substance FD-838 that was obtained by measurement in a chloroform solution;

Fig. 2 shows the $^1$H-NMR spectrum of the physiologically active substance FD-838 that was obtained by measurement in $CDC\ell_3$ at 400 MHz; and

Fig. 3 shows the $^{13}$C-NMR spectrum of the physiolo gically active substance FD-838 that was obtained by measurement in $CDC\ell_3$ at 100 MHz.

### DETAILED DESCRIPTION OF THE INVENTION

The strain used in the present invention to produce FD-838 has the following mycological features.

1. Morphology

This strain grows well on a variety of culture media such as a malt soup agar medium, a potato-glucose agar medium and a $Y_pS_8$ agar medium, with satisfactory bearing of conidia (phialo-type) being observed.

Microscopic observation of colonies appearing in a potato-glucose agar medium shows the following: the hyphae are clear, septate and are highly branched; the branched conidiophores arise from the substrate mycelium, are varied in size ranging from 150 to 300 μm in length and from 2.5 to 6.5 μm in diameter, and have smooth walls; the vesicles are flask-shaped and varied in diameter ranging from 15 to 25 μm, with parallel phialides typically forming at positions about one half the way along the length of the vesicles; the phialides range from 6.5 to 9.0 μm in length and from 2.0 to 2.2 μm in diameter, with no metula formation observed; one phialophore first bears at the tip of a phialide and, as the cultivation proceeds, becomes chain-like to form dense cylindrical conidial heads ranging from 120 to 150 μm in length; under observation with a microscope, the conidia range from spherical to subspherical in shape and from 2.4 to 3.0 μm in size, with burr-like pricks present on the surface.

2. Culture Properties

The strain was cultured on various media at 30°C for 14 days and the results of macroscopic observation of the cultures were as follows.

| Media | Growth on Media | Color of Reverse Side of Colonies | Conidia | | Soluble Pigment |
|---|---|---|---|---|---|
| | | | Formation | Color | |
| Malt soup agar medium | good; overall growth of velvety hyphae | pale brown | good | blue yellow; became powdery after maturation | negative |
| Potato-glucose agar medium | good; overall growth of wooly hyphae | creamy | good | dark green | yellow green |
| Czapek agar medium | good; overall growth of white velvety hyphae | creamy; partly brown | good | blue yellow; became powdery after maturation | pale brown |
| Sabouraud's agar medium | good; overall growth of white velvety hyphae | pale brown | good | yellow brown | dark red brown |
| Oatmeal agar medium | good; overall growth of white velvety hyphae | creamy | good | dark green | negative |
| Synthetic Mucor agar medium | good; overall growth of white felt hyphae | creamy | good | yellow brown | negative |
| YpSs agar medium | good; overall growth of white velvety hyphae | creamy | good | dark green | negative |

All of the media tested were negative for the formation of sclerotia.

3. Physiological and Ecological Properties

    (1) Optimum growth conditions:

This strain grows best in a $Y_pS_s$ medium at pH 3 to 7 and a temperature of 25 to 37°C.

    (2) Growth range:

This strain is capable of growth in a $Y_pS_s$ medium at pH 2 to 10 and a temperature of 20 to 50°C.

    (3) Utilization of aerobic oxygen:

Aerobic.

The strain under discussion has the features listed above, and the results of morphological observation of this strain revealed that it belongs to the genus Aspergillus. Therefore, on the basis of the features shown above, the present inventors compared this strain with the many known strains reported in S. Udagawa and K. Tsubaki, ed., "Kinrui Zukan (An Atlas of Fungi)" 1978, and Raper and Fennell, "The Genus Aspergillus" with a view of identifying the strain. As a result, it was found that the characteristics of the strain in question are most similar to those exhibited by Aspergillus fumigatus Fresenius. Therefore, the present inventors concluded that the strain at issue is of the same species as Aspergillus fumigatus Fresenius and named it Aspergillus fumigatus Fresenius F-838 (Fermentation Research Institute Accession Number FERM P-8406: International Accession Number BP-1124 under Budapest Treaty).

The new physiologically active substance FD-838 can be produced from this strain by following procedures that are generally the same as those employed in producing common fermentation products. Namely, FD-838 may be obtained by cultivating the Aspergillus fumigatus Fresenius strain F-838 under aerobic conditions in a medium containing a variety of nutrient substances.

A liquid medium is chiefly used, with the carbon source being glucose, sucrose, molasses, starch, etc., which may be used either independently or in admixture. A suitable nitrogen source is polypeptone, soybean meal, yeast extract, etc., which may be used either independently or in admixture. Any organic substances and inorganic salts that assist in the growth of the Aspergillus fumigatus Fresenius strain F-838 and which promote the production of the physiologically active substance FD-838 may be added to the medium as required.

Adekanol, silicone and other appropriate defoaming agents may be employed.

Cultivation is advantageously carried out under aerobic conditions by a suitable method such as shake cultivation or cultivation with aeration and agitation. Cultivation generally continues for 2 to 5 days at pH 3 to 7 and at a temperature of 25 to 37°C, preferably for a period of 3 to 4 days at pH 6 to 7 and at a temperature of 28 to 30°C.

The physiologically active substance FD-838 produced by performing cultivation under the conditions described above may be isolated by following the common procedures for recovering fermentation products. Stated more specifically, the culture solution is separated by centrifugation or filtration after completion of the cultivation, absorbed on a suitable carrier such as Dia-Ion HP-20 (the trade name of Mitsubishi Chemical Industries Limited) and eluted with an appropriate eluant such as a lower alcohol or acetone; the eluted fractions of the physiologically active substance FD-838 are concentrated, dissolved in a water-insoluble solvent such as ethyl acetate, benzene or chloroform; concentrated to form a syrup, which is redissolved in an organic solvent such as benzene, ethyl acetate or methanol, and subjected to column chromatography on a silica gel (e.g., Kieselgel 60 of Merck, West Germany) and gel filtration with Sephadex LH-20 (the tradename of Pharmacia Fine Chemicals), thereby isolating the physiologically active substance FD-838 in a pure form.

The physiologically active substance FD-838 obtained by the procedures described above has the following physiochemical properties.

    (1) Appearance: pale green powder;

    (2) Melting point: 98 to 99°C;

    (3) Elemental analysis:

| Found | Calculated |
|---|---|
| C: 64.20% | C: 64.23% |
| H: 5.36% | H: 5.14% |
| N: 3.39% | N: 3.41% |

    (4) Molecular weight: high-resolution mass spectrum

Found: 411.1356

Calculated: 411.1318;

    (5) Molecular formula: $C_{22}H_{21}NO_7$;

    (6) $[\alpha]_D^{26}$ : 0° (C = 0.1 in methanol Sol.);

    (7) UV absorption: measured in a methanol solution - 252 nm ($\varepsilon$ 7500), 283 nm ($\varepsilon$ 4110), 336 nm ($\varepsilon$ 10007);

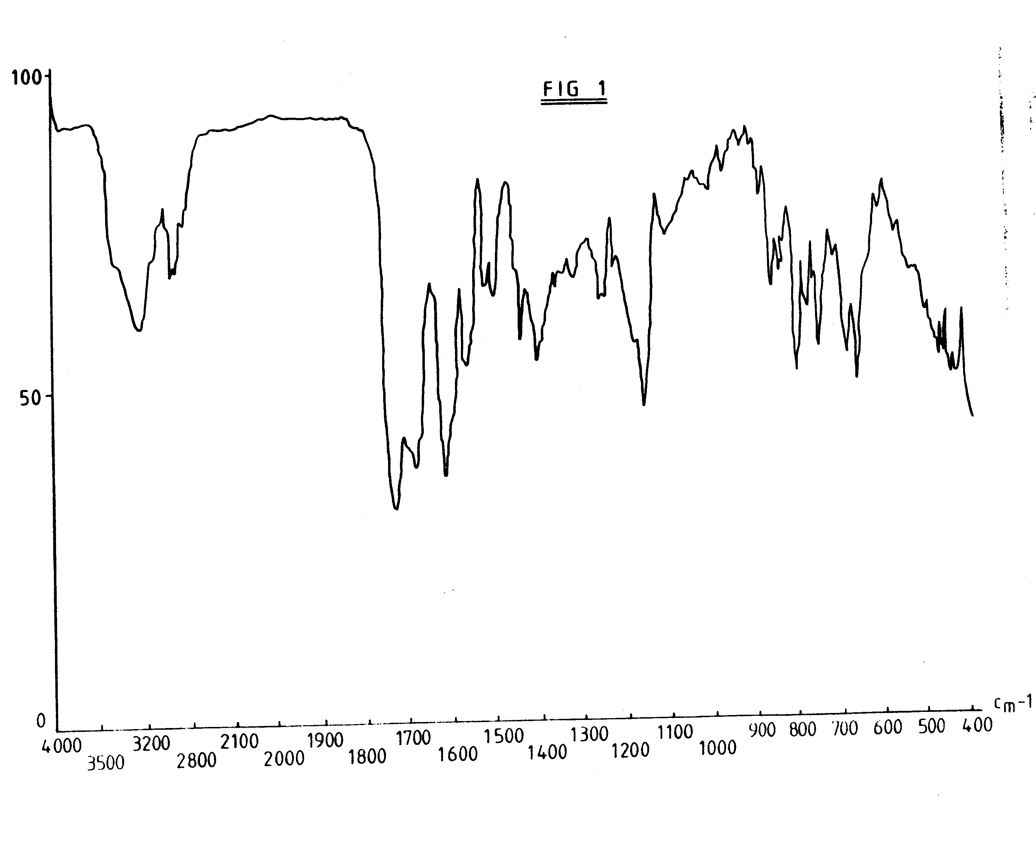

    (8) IR absorption spectrum: A spectrum obtained by measurement in chloroform solution is depicted in Fig. 1;

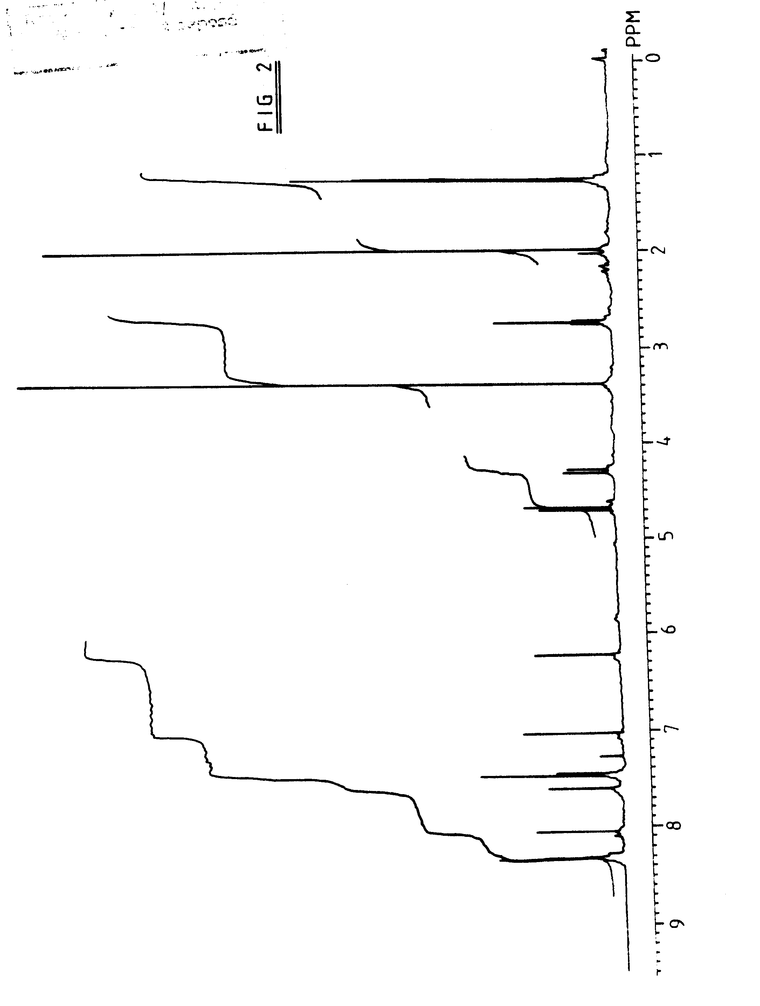

(9) $^1$H-NMR spectrum: A spectrum obtained by measurement in CDCl$_3$ at 400 MHz is depicted in Fig. 2;

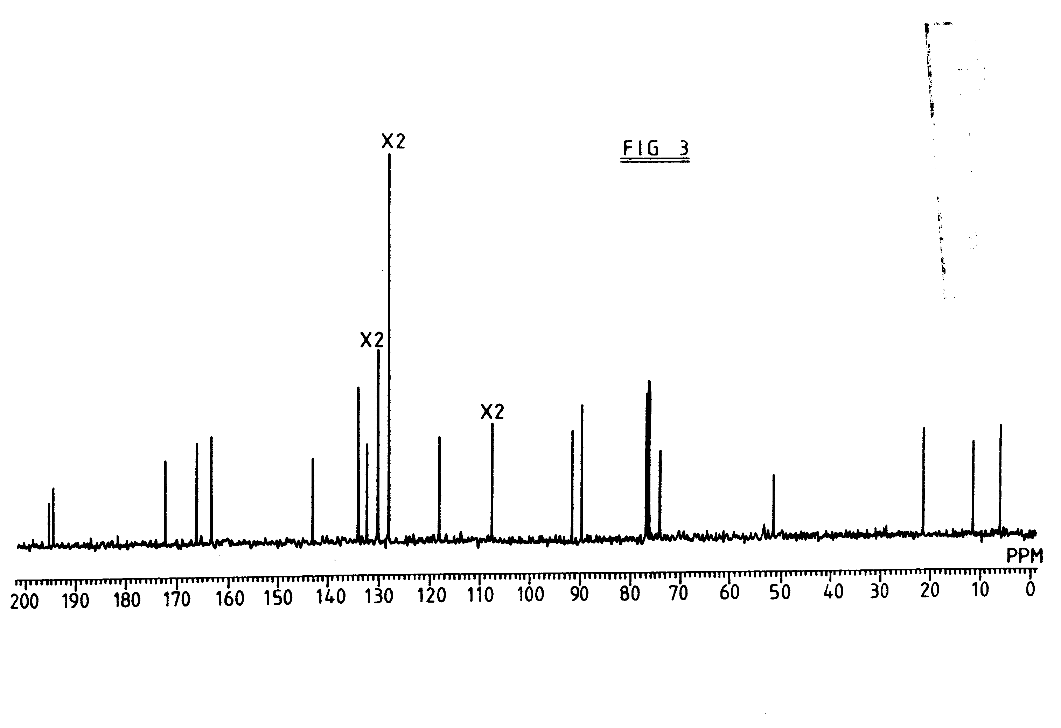

(10) $^{13}$C-NMR spectrum: A spectrum obtained by measurement in a CDCl$_3$ at 100 MHz is depicted in Fig. 3;

(11) Solubility:

Soluble in chloroform, benzene, methanol, ethanol, acetone and ethyl acetate;

Poorly soluble in ethyl ether, n-hexane and petro leum ether;

Insoluble in water;

(12) Color reaction:

Positive with sulfuric acid, iodide, anisaldehyde-H$_2$SO$_4$ and vaniline-H$_2$SO$_4$;

Negative with ninhydrin and BCG;

(13) Nature:

Basic (neither acidic nor neutral).

Capabilities of FD-838

The physiologically active substance FD-838 is capable of inducing the differentiation of leukemic cells and inhibiting the growth of certain Gram-positive bacteria and true fungi, as specifically demonstrated by the following Test Examples 1 and 2.

TEST EXAMPLE 1

Two-milliliter portions of an RPMI-1640 medium (Gibco) containing 15% fetal calf serum (Gibco) were put into Petri dishes 35 mm in diameter. The media was inoculated with $2 \times 10^5$ human promyelocytic leukemia cells (HL-60) per ml and cultivation was carried out for 3 days at 37°C in a 5% CO$_2$ incubator. FD-838 was added to the media simultaneously with start of the cultivation of HL-60 and the percentage of cells that were found positive for induction of differentiation by an NBT reduction test that was conducted in accordance with the method of Corrado et al. described in Cancer Research, 42, 445-449, 1982 was calculated. The same test was conducted at varying concentrations of FD-838 and the results are shown below in terms of the percentage of HL-60 that turned positive with NBT.

| FD-838 (μg/ml) | NBT Positive Cells (%) |
|---|---|
| 0 | 2.4 |
| 0.625 | 5.0 |
| 1.25 | 25 |
| 2.5 | 42 |
| 5.0 | 65 |

TEST EXAMPLE 2

Bacteria were cultivated in a heart infusion medium while a true fungus was cultivated in a Sabouraud's agar medium. For each of the microorganisms tested, the inoculum size was $10^6$ CFU/ml and the antimicrobial activity of FD-838 was determined by the paper disk method. The results are shown below.

| Microorganisms | Inhibition Zone (mm) | | |
|---|---|---|---|
| | 100 (μg/ml) | 50 (μg/ml) | 25 (μg/ml) |
| Staphylococcus aureus FDA209P | - | - | - |
| Escherichia coli NIHJ | - | - | - |
| Bacillus subtilis ATCC 6633 | - | - | - |
| Micrococcus luteus NIHJ | 12.1 | 11.0 | - |
| Candida albicans | 13.5 | 10.9 | - |

Having the characteristics described above, the physiologically active substance FD-838 which is the end substance of the present invention is not only useful to induce differentiation of leukemic cells in culture and inhibiting growth of certain Gram-positive bacteria and true fungi, but the substance is also useful as a medicine. For these medical purposes, this compound may be administered by injection (e.g., intravenously) in conventional dosage forms such as solutions, suspensions and the like, all of which are prepared according to conventional pharmaceutical practices.

The effective dosage of this compound depends on the age, weight or response of patient. Generally, however, a daily dosage for an average size adult human may range from 1 mg to 100 mg/one person.

The following example is given for the purpose of further illustrating the present invention but is in no sense to be taken as limiting.

EXAMPLE

(1) A sterile liquid medium (pH 7) containing 5% glucose and 1% polypeptone was inoculated with the Aspergillus fumigatus Fresenius strain F-838 and shake cultivation was conducted for 96 hours with agitation at 30°C to prepare a seed culture. Two liters of this seed culture was inoculated in 120 liters of a sterile medium in a fermentation tank (inner capacity, 200 ℓ) having the same composition as the seed culture, and fermentation was conducted for 72 hours at 30°C with aeration and agitation.

(2) After completion of the fermentation, the culture solution was filtered to obtain the cells and the filtrate, the latter being adsorbed on 5 ℓ of Dia-Ion Hp-20 and eluted with 10 ℓ of a 75% methanol solution. The eluate was concentrated to a volume of 4 ℓ. The concentrate was adjusted to a pH of 10 with 5 N NaOH and extracted twice with an equal volume of benzene. The benzene fractions were combined. dehydrated with anhydrous sodium sulfate, and concentrated to obtain a brown syrup.

(3) The syrup was dissolved in 50 ml of chloroform and adsorbed on a column packed with 300 ml of chloroform-equilibrated silica gel (Kieselgel 60 of Merck, West Germany). The column was eluted first with 600 ml of chloroform, then with 600 ml of a mixture of chloroform and methanol (99.8:0.2). The fractions were combined and concentrated to dryness, thereby obtaining 1.2 g of a crude powder.

(4) The crude powder obtained in (3) was dissolved in 5 ml of chloroform and adsorbed on a column packed with 50 ml of a n-hexane equilibrated silica gel (Kieselgel 60 of Merck, West Germany). The column was eluted with a mixture of n-hexane and acetone (85:15), and the fractions were concentrated to dryness to obtain 700 mg of a pale green powder.

This powder was dissolved in 3 ml of methanol and subjected to gel filtration with methanol on a column packed with 500 ml of Sephadex LH-20 (Pharmacia Fine Chemicals). The active fractions obtained were combined and concentrated to dryness to obtain about 600 mg of a pale green powder. This powder was found to have a melting point of 98 to 99°C.

While the invention has been described in detail and with reference to specific embodiments thereof, it will be apparent to one skilled in the art that various changes and modifications can be made therein without departing from the spirit and scope thereof.

Claims

1. A physiologically active substance FD-838 having the following physiochemical properties:
(1) Appearance: pale green powder;
(2) Melting point: 98 to 99°C;
(3) Elemental analysis: C 64.20%
H 5.36%
N 3.39%;
(4) Molecular weight: high-resolution mass spectrum

6

Found: 411.1356
Calculated: 411.1318;

   (5) Molecular formula: $C_{22}H_{21}NO_7$;
   (6) $[\alpha]_D^{26}$ : 0° (C = 0.1 in methanol sol.);
   (7) UV absorption: measured in a methanol solution
252 nm ($\varepsilon$ 7500), 283 nm ($\varepsilon$ 4110), 336 nm ($\varepsilon$ 10007);
   (8) IR absorption spectrum:
as shown in Fig. 1 (measured in chloroform sol.);
   (9) $^1$H-NMR spectrum:
as shown in Fig. 2 (measured in CDCl$_3$ at 400 MHz);
   (10) $^{13}$C-NMR spectrum:
as shown in Fig. 3 (measured in CDCl$_3$ at 100 MHz);
   (11) Solubility:
soluble in chloroform. benzene. methanol, ethanol, acetone and ethyl acetate;
poorly soluble in ethyl ether, n-hexane and petro leum ether;
insoluble in water;
   (12) Color reaction:
positive with sulfuric acid, iodide, anisaldehyde-$H_2SO_4$ and vaniline-$H_2SO_4$;
negative with ninhydrin and BCG;
   (13) Nature:
basic (neither acidic nor neutral).

2. A process for preparing a physiologically active substance FD-838 which comprises cultivating a physiologically active substance FD-838 producing microorganism of genus Aspergillus, and collecting said physiologically active substance FD-838 that has accumulated in the culture of said microorganism.

3. A process as in claim 2, wherein the physiologically active substance FD-838 producing microorganism is of the species Aspergillus fumigatus Fresenius.

4. A process as in claim 3, wherein the physiologically active substance FD-838 producing microorganism is Aspergillus fumigatus Fresenius F-838.

5. A process as in claim 4, wherein the physiologically active substance FD-838 producing microorganism is Aspergillus fumigatus Fresenius F-838 having Fermentation Research Institute Accession No. FERM P-8406 and International Accession No. BP 1124.


Claims for contracting state : AT

1. A process for preparing a physiologically active substance FD-838 which comprises cultivating a physiologically active substance FD-838 producing microorganism of genus Aspergillus, and collecting said physiologically active substance FD-838 that has accumulated in the culture of said microorganism.

2. A process as in claim 1, wherein the physiologically active substance FD-838 prcducing microorganism is of the species Aspergillus fumigatus Fresenius.

3. A process as in claim 2, wherein the physiologically active substance FD-838 producing microorganism is Aspergillus fumigatus Fresenius F-838.

4. A process as in claim 3, wherein the physiologically active substance FD-838 producing microorganism is Aspergillus fumigatus Fresenius F-838 having Fermentation Research Institute Accession No. FERM P-8406 and International Accession No. BP 1124.

FIG 1

FIG 2

FIG 3